# EUROPEAN PATENT APPLICATION

(11) **EP 2 896 964 A1**
(43) Date of publication of application: **22.07.2015**
(21) Application number: 14151421.6
(22) Date of filing: 16.01.2014
(51) Int. Cl.: G01N 33/68

(54) **MD-2 as biomarker in heart failure**

(71) Applicant: Ernst-Moritz-Arndt-Universität Greifswald, 17487 Greifswald (DE)
(72) Inventor: Riad, Alexander, 17489 Greifswald (DE)
(74) Representative: Wablat Lange Karthaus

(57) **Abstract**

Myeloid differentiation factor-2 (MD-2) has been shown to play role as risk predictor in heart failure, for example, Dilated Cardiomyopathy (DCM). The invention is directed to a method for determining the risk of mortality in a proband, especially a heart failure proband, characterized in that the proband's level of myeloid differentiation factor 2 (MD-2) is determined and in case of a higher MD-2 level a higher risk of mortality is diagnosed. Combined analysis of MD-2 and nt-pro BNP in significantly increased capability of risk prediction when compared to the nt-pro BNP analysis alone.

## Description

The present invention is related to a method for determining the risk of mortality in a proband (risk stratification), especially a heart failure proband, based on myeloid differentiation factor 2.

Dilated cardiomyopathy (DCM) is the most common cause of heart transplantation and constitutes the third most common type of heart failure [1]. This disease is characterized by ventricular chamber enlargement and systolic dysfunction. Although the overall outcome of those patients has been shown to be favorable, a significant proportion shows increased mortality [1]. The reason for the high variation regarding the outcome of patients with DCM is unknown. Thus, there is a growing interest for establishing biomarkers to obtain pathophysiological insight into and to possibly improve the management of patients with heart failure [2]. Current research regarding heart failure biomarkers focuses mainly on patients with coronary disease. Although risk stratification of DCM patients would particular desirable because of the high variation of the disease, only few disease specific investigations regarding biomarkers as potential surrogate parameters have been published. As recently shown, two inflammatory marker, cystatin C and syndecan-4, correlate with the surrogate parameter kidney function, exercise capacity and left ventricular (LV) remodelling, respectively, in DCM patients [3]. Another recently published study demonstrated that serial measurement of brain natriuretic peptide (BNP) and cardiac magnetic resonance predict LV reverse remodelling [4]. The inventors showed previously, that in DCM patients increased amounts of circulating leucocytes was significantly and independently from other cardiovascular risk factors associated with increased mortality [5]. Importantly, this was only the case for patients with severe LV dilation, whereas patients showing moderate or no LV dilation, leucocytes amount did not predict mortality.

General in heart failure (HF), brain natriuretic peptide (BNP) and its n-terminal region nt-pro BNP are widely accepted as gold standard diagnostic tools regarding among others risk stratification [6, 7]. BNP as well as nt-pro BNP are well-known and disclosed in several data bases (see, for example, BNP GenBank: BAA04067.1). proBNP is a protein with 1-108 amino acids (aa), wherein nt-proBNP has aa 1-76 of proBNP and BNP has aa 77-108 of proBNP. In the Val-HeFT (Valsartan Heart Failure Trial) BNP predicted morbidity and mortality significantly [8]. A pooled review showed a 35% increase in relative risk of death for each 100-pg/mL increment in baseline BNP [9, 10]. BNP has been shown to predict other important end points, including hospitalization risk in patients presenting with heart failure [11] and likelihood of rehospitalisation based on predischarge value [12]. However, although BNP has been tested in large controlled heart failure trials, there is no study investigating the impact of BNP exclusively in patients with DCM. DCM is associated with a chronic inflammatory state, with the direction of causation being an active area of debate [10]. Other relevant types of heart failure beside DCM are ischemic cardiomyopathy, hypertrophic cardiomyopathy, inflammatory cardiomyopathy (myocarditis) and toxic cardiomyopathy.

There is still a need to find new biomarkers, which help to analyse the status of a proband having heart failure and for risk stratification to determine the proband's mortality risk and need for preventive intervention.

Thus, the objective of the present invention was the provision of new biomarkers for heart failure probands.

The problem was solved by a method according to claim 1 and claim 9 as well as by a kit according to claim 10. Preferred embodiments are disclosed in the dependent claims.

In other words, the problem was solved by a method for determining the risk of mortality in a proband, especially a heart failure proband, characterized in that the proband's level of myeloid differentiation factor 2 (MD-2) is determined and in case of a higher MD-2 level a higher risk of mortality is diagnosed. Using multivariate analysis, this was independently from other risk factors.

In the following, the terms "proband" and "patient" were used synonymously.

The patient's diagnose of being a heart failure proband is done initially by methods known in the art, for example, by echocardiography or analysis of specific biomarkers etc..

MD-2 has been shown to play an important role in regulating the innate immune system, but its role in cardiac diseases was so far unknown. MD-2 is a 18-25-kDa protein that is both bound to a receptor protein of the innate immune system, namely Toll-like receptor 4 (TLR4) and is secreted as a soluble molecule from MD-2-expressing cells [13]. It is a well-known protein, which is disclosed in several data bases including DNA and protein sequences (see for example, MD-2 homo sapiens, protein: GenBank: BAG55275.1; mRNA: GenBank: AB446498.1). MD-2 has been shown to enhance the response to lipopolysaccharide (LPS) in Toll-like receptor 4 (TLR4)-transfected HEK293 cells [14]. In addition, MD-2 knockout mice do not respond to LPS [15]. The role of MD-2 in cardiac diseases including HF and DCM was unknown so far.

The MD-2 level is determined in vitro based on body material samples, especially body fluid samples or body tissue samples. The body fluid samples are samples of blood plasma, blood serum and/or cerebrospinal fluid, preferably blood serum samples.

The use of the method is focused on eucariotes, preferably selected from human or animal, most preferred is a human proband. The MD-2 is analyzed on protein level or on nucleic acid level, preferably on protein level. In other words, the MD-2 protein contents of body fluids, for example, blood serum, are measured, for example, by ELISA methods.

In a preferred embodiment, the reference value when diagnosing heart failure patient's risk of mortality on the protein level in blood serum samples is about 300 ±30 ng/ml. This is the overall median, which was determined on clinical basis (302.44 ng/ml, see below). The more a patient's MD-2 level exceeds this reference value, the higher is his risk of mortality. This means that a MD-2 level increase of 10 ng/ml leads to mortality risk increase of 5%, increase of 100 ng/ml already leads to a mortality risk increase of 65%. Per 1000 ng/ml, mortality risk increased 147-fold.

Said reference value of 300 ±30 ng/ml (protein in blood serum sample) was determined especially for DCM but applies also for other heart failure diseases.

In a special embodiment, the method for determining the risk of mortality in a proband, especially a heart failure proband, by determining his MD-2-level is combined with an analysis of the the BNP level and/or the nt-pro BNP level. This/these level(s) is/are determined and in case of MD-2 level and BNP level and/or nt-pro BNP level being higher a higher risk of mortality is diagnosed. This means that the MD-2 level is determined in any case and additionally, either the BNP or the nt-proBNP level or both are measured and considered.

In one preferred embodiment, the nt-pro BNP level is measured and the results are combined with the MD-2 level determination in order to determine a patient's risk of mortality.

The BNP- and/or nt-pro BNP-level is/are also determined in vitro based on body material samples, especially body fluid samples or body tissue samples as outlined for MD-2 above.

The above-described methods are suitable to determine the risk stratification in heart failure patients. However, the MD-2-level can also be used for diagnosing or pre-diagnosing a heart failure or for determining the risk of a proband to develop a heart failure. In other words, a special embodiment of the invention is directed to a method for diagnosing or pre-diagnosing a heart failure or for determining the risk of a proband to develop a heart failure disease, characterized in that the proband's level of MD-2 is determined and compared to a MD-2 level which is associated with no heart failure (of a healthy proband), whereas in case of a higher MD-2 level heart failure or an increased risk of developing a heart failure is diagnosed/pre-diagnosed.

In this case, the MD-2 level is also determined as outlined above regarding the risk stratification, i.e. it is determined in vitro based on body material samples, especially body fluid samples or body tissue samples, more preferably samples of blood plasma, blood serum and/or cerebrospinal fluid, most preferably blood serum samples. It can be evaluated on protein level or on nucleic acid level, preferably on protein level. This applies all for the proband under consideration as well as for the healthy proband, who's MD-2 level serves as the reference value.

Another specific embodiment of the invention is directed to a diagnostic kit for carrying out one of the methods described above. The kit is preferably based on an Enzyme Linked Immunosorbent Assay (ELISA) test system and comprises an antibody, which is capable of detecting MD-2 protein. If the analysis is done on the nucleic acid level, the kit is for example an RNA detection kit.

Regarding the present invention, the role of MD-2 was initially investigated for heart failure diseases on the example of DCM, i.e. in a patient cohort with biopsy-guided DCM. However, even if DCM was initially investigated, the invention is not restricted to DCM but rather directed to all heart failure diseases as outlined above, DCM is just one preferred embodiment. Other heart failures beside DCM, which are also relevant, are other cardiomyopathies such as ischemic cardiomyopathy, hypertrophic cardiomyopathy, inflammatory cardiomyopathy (myocarditis) and toxic cardiomyopathy.

The DCM-based investigation is described below:

### Materials and methods

### Clinical study

### Study population

A total of 174 patients with DCM was included during 2005 and 2011. Inclusion criteria of DCM were increased left ventricular end-diastolic diameter (LVEDD >58 mm) and reduced ejection fraction (LVEF <50%) as derived by echocardiography. In all patients significant coronary artery disease or heart-valve diseases were ruled out by angiography and echocardiography, respectively, as described previously [5, 16]. In all patients the vital status was known (with a minimum follow-up time of three month for living patients). All patients gave informed and written consent. The study was approved by the local ethics committee of the University Medicine Greifswald, Germany.

### Study design and end points

An unmatched case-control study was performed. All cases (n=34) and non-cases (=controls, n=140) where both nt-proBNP and MD-2 blood levels were determined were taken. In total 174 patients were included in the analysis. A Kaplan-Meier survival analysis and a multivariate Cox regression were performed to test whether MD-2 is a predictor for mortality in these patients. Based on sex-and age-adjusted Cox models, ROC analyses were performed to compare the discrimination capability of nt-proBNP and MD-2. As sensitivity analysis this initial dataset was used and two new datasets by a 1:2 and 1:3 matching according to sex and age (5 year radius), respectively were created. In the first new dataset 1 case was lost due to missing of appropriate controls and in the second dataset 8 cases were lost, respectively. So the first dataset consisted of 99 patients (33 cases, 66 controls) and the second one of 104 patients (26 cases, 78 controls). On these two datasets the ROC analysis was repeated and the AUC change was tested again. Study population has been in part investigated previously [5, 16]. Patients received heart failure medical treatment leaned to the current guidelines of the european society of cardiology [7]. Median follow-up was 3.37 years (2.12;4.45). The primary end point of this study was all-cause mortality. Information on vital status of patients was obtained from official resident data files in May 2011. Subjects were censored either at last known date of contact (either a clinic visit or date of information from the population registry) or at death. Furthermore, the discrimination power of MD-2 and nt-pro-BNP regarding the prediction of mortality was compared.

### Echocardiography

As described previously [5, 16], echocardiographic parameters were performed in all patients by 2-dimensional echocardiography at first hospital admission according to the American College of Cardiology/American Heart Association guidelines [17]. Left ventricular ejection fraction was quantified according to the biplane Simpson methodology. Left ventricular dilation was assessed by measurement of the LVEDD.

### Endomyocardial biopsies

EMBs from the right ventricular septum were performed as previously described [5]. In brief, right jugular vein access was used to introduce a guiding sheath (7F St. Jude Medical). Biopsy specimens were taken using a disposable dedicated cardiac biotome (7F 50 cm, Ref 502-402B, Cordis, The Netherlands) under biplane fluoroscopic control (40 ° LAO and 90 ° LAO), which helps guide the tip of the catheter to the right ventricular septum.

### Analyses of endomyocardial biopsies

Histopathological and immunohistochemical analyses of endomyocardial biopsies were performed as previously described [5, 18]. Immunohistological analyses were used to investigate cardiac inflammation by treating the paraffin embedded tissue sections with an avidinbiotin-immunoperoxidase method according to the manufacturer's protocol (Vectastatin Elite ABC kit, Vectastatin®, USA). Monoclonal antibodies were used to evaluate cardiac cell infiltration of CD3+ T-lymphocytes (Novocastra laboratories, UK), and CD68+ macrophages according to the current guidelines for the Definition and Classification of Cardiomyopathies [19].

### Detection of Viral Genomes

As described previously [18], Enterovirus species (comprising coxsackieviruses and echoviruses), parvovirus B19, adenoviruses, Epstein-Barr virus, and human herpesvirus type 6 were evaluated by nested PCR/RT-PCR from deep-frozen or RNAlater-fixed endomyocardial biopsy specimens. In brief, for RT-PCR analyses, RNA was transcribed into cDNA by RT according to the protocol of the manufacturer (AGS, Heidelberg, Germany). The enzymatic amplification of cDNA respectively DNA was performed as nested PCR on a Perkin-Elmer GeneAmp PCR System 9600 (Applied Biosystems, Weiterstadt, Germany) in two 30-cycle programs. As an internal control for successful isolation of nucleic acids, the housekeeping gene GAPDH was detected by PCR. A biopsy was considered positive for viral infection if viral genome was detected by PCR, and specificity was confirmed by automatic DNA sequencing of viral amplification products.

### Measurement of nt-pro brain natriuretic peptide and MD-2

Blood samples were obtained during the first admission in our hospital and recorded in the electronic case report form. Nt-pro brain natriuretic peptide (nt-pro BNP) levels were quantified according to routine diagnostic methods in the inventor's clinic as previously described [16]. MD-2 protein contents were measured in serum by ELISA (Loxo, Germany, No. 6E97705Hu) according to the manufacturers recommendations.

### Statistical analyses

All statistical analysis was done in STATA 12 (StataCorp. 2011, Release 12. College Station, TX: StataCorp LP). Baseline characteristics were compared either using the Fischer Exact test for categorigal variables or the Mann Withney U Test for continuous variables. For Kaplan-Meier survival curve analysis, patients were divided into two groups using the median of the MD-2 concentration. The log-rank test was used to compare Kaplan-Meier survival curves between both groups. All-cause mortality was also analysed using a multivariable Cox regression model with age, sex, BMI, systolic and diastolic blood pressure, LV-EF, LVEDD, Smoking and Diabetes as covariables. In order to compare the discrimination capability of nt-proBNP and MD-2 regarding the patients' vital status four age- and sex-adjusted Cox regression models were calculated: predictor variable = (1) only nt-proBNP; (2) only MD-2; (3) nt-proBNP and MD-2 as additive main effects; and (4) nt-proBNP and MD-2 as interactive effects. The area under the curve (AUC) was calculated as Harrel's c [42, 43] and tested for significant differences.

### Results

### Baseline characteristics according to white blood cell count

In total, 174 patients were included in the inventor's study. In all patients, blood MD-2 protein level was quantified at first hospital admission. Table 1 shows the baseline characteristics of the patients at first hospital admission in the hospital separated to blood MD-2 level ≤ or > of the overall median. Both patients' group displayed reduced systolic LV function indexed by impaired LV ejection fraction and LV dilation indexed by increased LVEDD. These two groups did not differ significantly regarding age, gender, LV ejection fraction, LVEDD and cardiovascular risk factors at first admission. In addition, heart-failure medication did not differ indexed by similar percent of the maximum dose of ß-blockers, ACE inhibitors, AT1 antagonists and aldosterone antagonists evaluated at first admission. In addition, presence of cardiac inflammation and virus genome did not differ between patients with MD-2 blood level ≤ overall median when compared to patients with MD-2 blood level > overall median.

### Impact of MD-2 on all-cause mortality

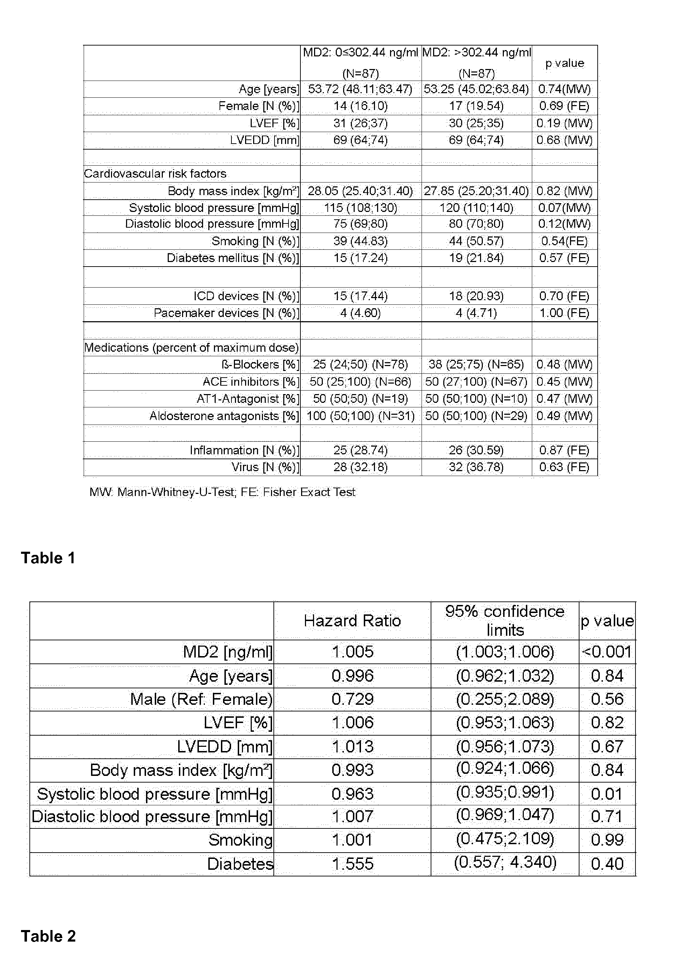

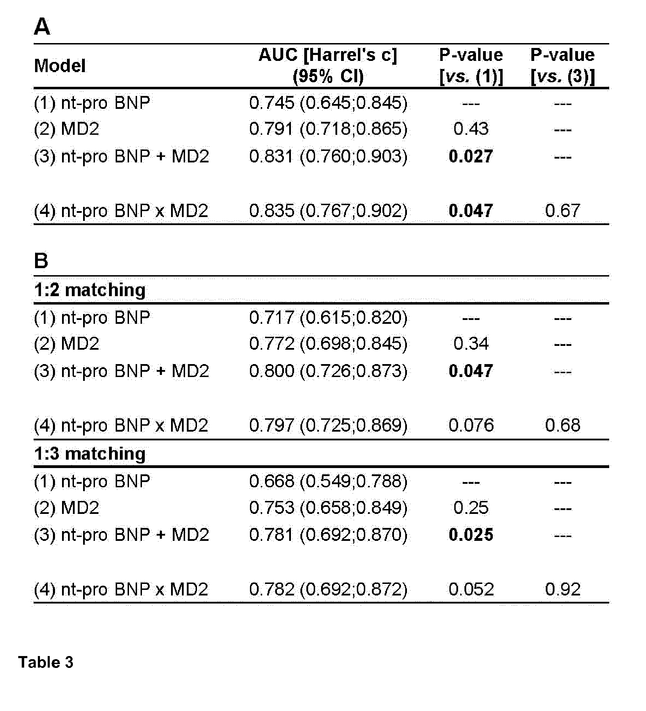

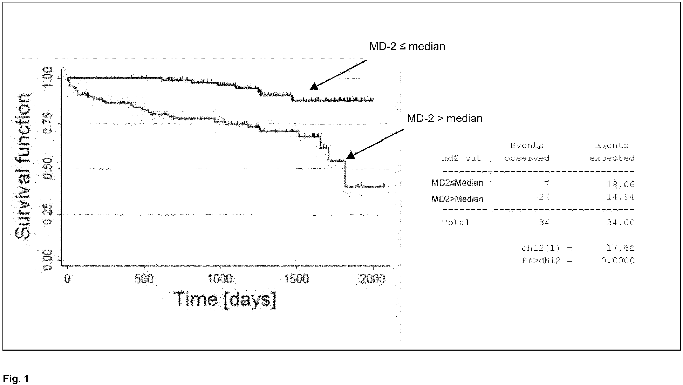

Figure 1 shows a Kaplan Meier curve of patients with DCM distributed by serum MD-2 level ≤ or > of the overall median quantified at first hospital admission in the hospital. Patients with MD-2 level higher than the median displayed significantly increased mortality when compared to Patients with MD-2 lower than the median (log-rank test: chi2 = 17.62, P<0.001). Absolute risk increase was 23% in patients with MD-2 level > of the overall median (31% absolute mortality) when compared to patients with MD-2 level ≤ of the overall median (8% absolute mortality).

### Multivariable cox regression analyses

Multivariable cox regression analyses in patients with DCM are shown in table 2. It showed that increased mortality was independent from age, gender, LV ejection fraction, LVEDD and other cardiovascular risk factors including diabetes, BMI, diastolic blood pressure and smoking. However, increased mortality was significantly dependent on MD-2 blood level with a hazard ratio of 1.005 (p<0.001) and systolic blood pressure with a hazard ration of 0.963 (p<0.01). These results indicated that a MD-2 level increase of 10 ng/ml leads to mortality risk increase of 5.1%, while an increase of 100 ng/ml already leads to a mortality risk increase of 64.7%.

### Mortality prediction by MD-2 and nt-pro BNP

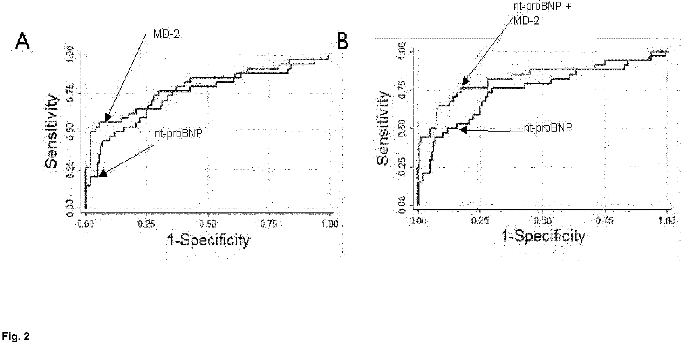

Figure 2a shows ROC curves for age- and sex-adjusted Cox Hazard models with either nt-pro BNP level or MD-2 level as predictors for mortality. The area under the curve (AUC) was non significantly higher for MD-2 when compared to nt-pro BNP (p=0.43) as shown in C (1) and (2). Figure 2b shows ROC curves for age- and sex-adjusted Cox Hazard models with either nt-pro BNP level or both nt-pro BNP combined with MD-2 level as predictors for mortality. AUC for the combination of MD-2 and nt-pro BNP was significantly increased when compared to the AUC for nt-pro BNP alone (p= 0.027) as also shown in table 3A (1) and (3). MD-2 in combination with nt-pro BNP improved sensitivity and specifity by 6% and 3%, respectively, when compared to nt-pro BNP alone. Sensitivity increases from 76% to 82% and specificity increases from 69% to 72%. As sensitivity analysis we repeated the ROC analyses for a case-control matched design of 1:2 and 1:3, respectively, in the study group. The resulting first dataset consisted of 99 patients (33 cases, 66 controls) and the second one of 104 patients (26 cases, 78 controls). Table 3B shows AUC values from these ROC analyses for MD-2 and nt-pro BNP alone, respectively, and the combination of MD-2 and nt-pro BNP. Results were similar when compared to the unmatched study population, thus the AUC differences between nt-pro BNP alone and the combination of nt-pro BNP and MD-2 seemed to be a reliable and stable effect.

### Discussion

For the first time an impact of the innate immune protein MD-2 on clinical outcome in patients with DCM was shown. The results indicate further, using MD-2 as a biomarker in addition to nt-pro BNP increases significantly the discrimination capability regarding prediction of mortality among these patients.

In carefully characterized patients with DCM, MD-2 protein level in peripheral blood quantified at first hospital admission predicts highly statistically significant long term mortality. Absolute risk increase in patients displaying MD-2 blood concentration greater than the overall median was 23% (P<0.0001). Multivariate Cox regression analyses demonstrated that mortality risk was highly dependent on MD-2 blood concentration with a hazard ratio of 1.005 (p<0.001). This means that a MD-2 level increase of 10 ng/ml leads to mortality risk increase of 5.1%, increase of 100 ng/ml already leads to a mortality risk increase of 64.7%. Per 1000 ng/ml, mortality risk increased 147-fold. Thus, in the light of measured MD-2 concentrations in the patients cohort between 131 and 1216 ng/ml, substantial impact of absolute risk alterations are observed depending on this immune protein measurement. To further investigate the clinical impact of MD-2 blood level, the capability of risk prediction in comparison to nt-pro BNP was analysed, which is widely accepted as the gold standard laboratory parameter for risk stratification in heart failure patients [7, 10]. ROC curve analyses of MD-2 and nt-pro BNP showed similar risk prediction capability indexed by non statistically difference between AUC of both. This demonstrates non-inferiority of MD-2 regarding risk prediction in DCM patients when compared to nt-pro BNP. Interestingly, AUC for the combination of MD-2 and nt-pro BNP was significantly increased when compared to the AUC for nt-pro BNP alone (p= 0.027). Using both parameters MD-2 and nt-pro BNP increases sensitivity by 6% and specificity by 3% when compared to nt-pro BNP alone. Notably, nt-pro BNP as a single

biomarker for risk in heart failure has already a high sensitivity and specificity as also shown in the inventor's study (76% and 69%, respectively) and under other conditions of heart failure [2, 10].
A limited number of studies suggest that systemic inflammation plays a pathogenetic key role regarding the development and the progression of heart failure including DCM [22]. For example, increased levels of inflammatory markers such as C-reactive protein, C3 and C4 complement fractions and adhesion molecules in DCM have been shown previously [23]. In addition, increased cytokine levels in DCM are well documented [22]. The cytokine tumor necrosis factor alpha has been shown to be widely distributed in the myocardium of DCM patients, but it plays no role as a prognostic marker [24]. In the inventor's study, they identified a new powerful marker for the clinical risk stratification of patients with DCM in addition to the well established nt-pro BNP.
MD-2 is known as a key protein substantially modulating LPS and TLR4 signalling pivotal in immune cells [15, 25]. In the case of TLR4, there is evidence for a potential role of this receptor in heart failure. It is the best characterized immune protein of the TLR family [8]. It is located in the cell membrane and is known to play a key role in recognising pathogen associated molecular patterns (PAMPs) and in initiating cytokine activation in a large number of cell types including cardiac cells [26]. Loss of TLR4 in knockout mice models showed protective effects in several types of heart failure, to include myocardial ischemia, pressure overload, viral myocarditis and toxic cardiomyopathy [27-30]. DCM patients showed impaired cardiac recovery when carrying two genetic variants (rs4986790, TLR4 c.1187A>G, p.299D>G and rs4986791,TLR4 c.1487C>T, p.T3991) [16]. However, the role of MD-2 in DCM and generally in heart failure was unknown so far.

Taken together, the inventors could demonstrate for the first time that in a carefully characterized patients cohort with DCM, the innate immune protein MD-2 is a powerful predictor for long term mortality and gives additional capability regarding risk prediction to nt-pro BNP.

### Clinical perspective

Biomarkers are widely used to either detect or manage diseases. In the case of heart failure, despite of intense current research, only few biomarkers are used in clinical routine [7]. In this regard, BNP plasma level is often used to differentiate heart failure from other causes of dyspnoe, especially upon presentation to the emergency room [39]. In addition, BNP plasma levels predict mortality and major cardiovascular events in heart failure patients and in the general population [40, 41]. Although high sensitivity and specifity of this cardiac-specific Marker [2, 7], in the present study a new biomarker was identified reflecting systemic inflammation, which even increases risk prediction additional to BNP in patients with DCM. Especially in this disease with high clinical variation, early detection of worse outcome constitutes a valuable diagnostic tool which might possibly improve the management of patients in future. Despite of risk prediction, performing new therapeutic strategies are essential in DCM since this disease has no solid evidenced causal therapy despite of general heart failure therapy. Thus, identifying new contributors to this disease may help to develop new therapeutic strategies.

### Legends

### Table 1: Baseline characteristics of patients with DCM

In total, 174 patients were included in the study. Table 1 shows the baseline characteristics (age, gender, cardiovascular risk factors and medication) distributed by MD-2 (MD2) protein expression level in whole blood at first hospital admission (≤ or > of overall median). Data are presented as median (1st quartile; 3rd quartile) and absolute number (proportion in %), respectively. Statistical analyses were performed using the Fisher Exact Test (FE) or the Mann-Whitney-U-Test (MW), respectively.
Table 2: Multivariable cox regression analysis
   Table 2 shows multivariable Cox regression analyses of the patients population adjusted to MD-2 (MD2), age, gender, LVEF, LVEDD, Body-mass-index (BMI), systolic (RRsyst) and diastolic (RRdiast) arterial blood pressure, diabetes and smoking. Data are presented as calculated Hazard ratio, p value and the 95% confidence limits.
Table 3: AUC values from ROC curve analyses for the discrimination capability of nt-proBNP alone and nt-proBNP combined with MD-2
   Table 3A shows AUC of nt-pro BNP, MD-2 (MD2), nt-pro BNP + MD-2 and nt-pro BNP x MD-2.
   Table 3B shows AUC from the same ROC analyses after 1:2 and 1:3 randomization, respectively, in the study population. Statistical analysis was performed using the Harrel's c test.

Figure 1: Survival of DCM patients distributed by MD-2 level
   Figure 1 showing the Kaplan-Meier curves of patients with DCM during observation period of median 3.37 years distributed by the overall median of plasma MD-2 protein level quantified at first hospital admission.
Figure 2: ROC curve analyses for the discrimination capability of nt-proBNP alone and nt-proBNP combined with MD-2
   Figure 2 shows ROC curve analyses of plasma MD-2 versus nt-pro BNP level (A) and MD-2 in conjunction with nt-pro BNP level versus nt-pro BNP level alone.

### References

1. Mcnamara D M, Starling R C, Cooper L T, Boehmer J P, Mather P J, Janosko K M, Gorcsan J, 3rd, Kip K E,Dec G W. Clinical and Demographic Predictors of Outcomes in Recent Onset Dilated Cardiomyopathy Results of the IMAC (Intervention in Myocarditis and Acute Cardiomyopathy)-2 Study. J Am Coll Cardiol. 2011; 58(11):1112-8.
2. Braunwald E. Biomarkers in heart failure. N Engl J Med. 2008; 358(20):2148-59.
3. Bielecka-Dabrowa A, Von Haehling S, Aronow W S, Ahmed M I, Rysz J,Banach M. Heart failure biomarkers in patients with dilated cardiomyopathy. Int J Cardiol. 2013.
4. Kubanek M, Sramko M, Maluskova J, Kautznerova D, Weichet J, Lupinek P, Vrbska J, Malek I,Kautzner J. Novel predictors of left ventricular reverse remodeling in individuals with recent-onset dilated cardiomyopathy. J Am Coll Cardiol. 2013; 61(1):54-63.
5. Riad A, Weitmann K, Herda L R, Empen K, Gross S, Nauck M, Dorr M, Klingel K, Kandolf R, Hoffmann W,Felix S B. Initial white blood cell count is an independent risk factor for survival in patients with dilated cardiomyopathy. Int J Cardiol. 2012.
6. Januzzi J L,Troughton R. Are serial BNP measurements useful in heart failure management? Serial natriuretic peptide measurements are useful in heart failure management. Circulation. 2013; 127(4):500-7; discussion 508.
7. Mcmurray J J, Adamopoulos S, Anker S D, Auricchio A, Bohm M, Dickstein K, Falk V, Filippatos G, Fonseca C, Gomez-Sanchez M A, Jaarsma T, Kober L, Lip G Y, Maggioni A P, Parkhomenko A, Pieske B M, Popescu B A, Ronnevik P K, Rutten F H, Schwitter J, Seferovic P, Stepinska J, Trindade P T, Voors A A, Zannad F,Zeiher A. ESC Guidelines for the diagnosis and treatment of acute and chronic heart failure 2012: The Task Force for the Diagnosis and Treatment of Acute and Chronic Heart Failure 2012 of the European Society of Cardiology. Developed in collaboration with the Heart Failure Association (HFA) of the ESC. Eur Heart J. 2012; 33(14):1787-847.
8. Latini R, Masson S, Anand I, Salio M, Hester A, Judd D, Barlera S, Maggioni A P, Tognoni G,Cohn J N. The comparative prognostic value of plasma neurohormones at baseline in patients with heart failure enrolled in Val-HeFT. Eur Heart J. 2004; 25(4):292-9.
9. Doust J A, Pietrzak E, Dobson A,Glasziou P. How well does B-type natriuretic peptide predict death and cardiac events in patients with heart failure: systematic review. BMJ. 2005; 330(7492):625.
10. Ketchum E S,Levy W C. Establishing prognosis in heart failure: a multimarker approach. Prog Cardiovasc Dis. 2011; 54(2):86-96.
11. Maisel A, Hollander J E, Guss D, Mccullough P, Nowak R, Green G, Saltzberg M, Ellison S R, Bhalla M A, Bhalla V, Clopton P,Jesse R. Primary results of the Rapid Emergency Department Heart Failure Outpatient Trial (REDHOT). A multicenter study of B-type natriuretic peptide levels, emergency department decision making, and outcomes in patients presenting with shortness of breath. J Am Coll Cardiol. 2004; 44(6):1328-33.
12. Verdiani V, Nozzoli C, Bacci F, Cecchin A, Rutili M S, Paladini S,Olivotto I. Pre-discharge B-type natriuretic peptide predicts early recurrence of decompensated heart failure in patients admitted to a general medical unit. Eur J Heart Fail. 2005; 7(4):566-71.
13. Jerala R. Structural biology of the LPS recognition. Int J Med Microbiol. 2007; 297(5):353-63.
14. Yang H, Young D W, Gusovsky F,Chow J C. Cellular events mediated by lipopolysaccharide-stimulated toll-like receptor 4. MD-2 is required for activation of mitogen-activated protein kinases and Elk-1. J Biol Chem. 2000; 275(27):20861-6.
15. Nagai Y, Akashi S, Nagafuku M, Ogata M, Iwakura Y, Akira S, Kitamura T, Kosugi A, Kimoto M,Miyake K. Essential role of MD-2 in LPS responsiveness and TLR4 distribution. Nat Immunol. 2002; 3(7):667-72.
16. Riad A, Meyer Zu Schwabedissen H, Weitmann K, Herda L R, Dorr M, Empen K, Kieback A, Hummel A, Reinthaler M, Grube M, Klingel K, Nauck M, Kandolf R, Hoffmann W, Kroemer H K,Felix S B. Variants of Toll-like receptor 4 predict cardiac recovery in patients with dilated cardiomyopathy. J Biol Chem. 2012.
17. Cheitlin M D, Alpert J S, Armstrong W F, Aurigemma G P, Beller G A, Bierman F Z, Davidson T W, Davis J L, Douglas P S,Gillam L D. ACC/AHA Guidelines for the Clinical Application of Echocardiography. A report of the American College of Cardiology/American Heart Association Task Force on Practice Guidelines (Committee on Clinical Application of Echocardiography). Developed in collaboration with the American Society of Echocardiography. Circulation. 1997; 95(6):1686-744.
18. Kindermann I, Kindermann M, Kandolf R, Klingel K, Bultmann B, Muller T, Lindinger A,Bohm M. Predictors of outcome in patients with suspected myocarditis. Circulation. 2008; 118(6):639-48.
19. Maron B J, Towbin J A, Thiene G, Antzelevitch C, Corrado D, Arnett D, Moss A J, Seidman C E,Young J B. Contemporary definitions and classification of the cardiomyopathies: an American Heart Association Scientific Statement from the Council on Clinical Cardiology, Heart Failure and Transplantation Committee; Quality of Care and Outcomes Research and Functional Genomics and Translational Biology Interdisciplinary Working Groups; and Council on Epidemiology and Prevention. Circulation. 2006; 113(14):1807-16.
20. Landsberger M, Staudt A, Choudhury S, Trimpert C, Herda L R, Klingel K, Kandolf R, Schultheiss H P, Kroemer H K, Volker U,Felix S B. Potential role of antibodies against cardiac Kv channel-interacting protein 2 in dilated cardiomyopathy. Am Heart J. 2008; 156(1):92-99e2.
21. Grube K, Rudebusch J, Xu Z, Bockenholt T, Methner C, Muller T, Cuello F, Zimmermann K, Yang X, Felix S B, Cohen M V, Downey J M,Krieg T. Evidence for an intracellular localization of the adenosine A2B receptor in rat cardiomyocytes. Basic Res Cardiol. 2011; 106(3):385-96.
22. Lappe J M, Pelfrey C M,Tang W H. Recent insights into the role of autoimmunity in idiopathic dilated cardiomyopathy. J Card Fail. 2008; 14(6):521-30.
23. Sampietro T, Neglia D, Bionda A, Dal Pino B, Bigazzi F, Puntoni M, Startari U, Morales A, Minichilli F, Bianchi F,L'abbate A. Inflammatory markers and serum lipids in idiopathic dilated cardiomyopathy. Am J Cardiol. 2005; 96(12):1718-20.
24. Zimmermann O, Kochs M, Zwaka T P, Bienek-Ziolkowski M, Hoher M, Hombach V,Torzewski J. Prognostic role of myocardial tumor necrosis factor-alpha and terminal complement complex expression in patients with dilated cardiomyopathy. Eur J Heart Fail. 2007; 9(1):51-4.
25. Park B S, Song D H, Kim H M, Choi B S, Lee H,Lee J O. The structural basis of lipopolysaccharide recognition by the TLR4-MD-2 complex. Nature. 2009; 458(7242):1191-5.
26. Akira S,Takeda K. Toll-like receptor signalling. Nat Rev Immunol. 2004; 4(7):499-511.
27. Riad A, Bien S, Gratz M, Escher F, Westermann D, Heimesaat M M, Bereswill S, Krieg T, Felix S B, Schultheiss H P, Kroemer H K,Tschope C. Toll-like receptor-4 deficiency attenuates doxorubicin-induced cardiomyopathy in mice. Eur J Heart Fail. 2008; 10(3):233-43.
28. Riad A, Jager S, Sobirey M, Escher F, Yaulema-Riss A, Westermann D, Karatas A, Heimesaat M M, Bereswill S, Dragun D, Pauschinger M, Schultheiss H P,Tschope C. Toll-like receptor-4 modulates survival by induction of left ventricular remodeling after myocardial infarction in mice. J Immunol. 2008; 180(10):6954-61.
29. Fuse K, Chan G, Liu Y, Gudgeon P, Husain M, Chen M, Yeh W C, Akira S,Liu P P. Myeloid differentiation factor-88 plays a crucial role in the pathogenesis of Coxsackievirus B3-induced myocarditis and influences type I interferon production. Circulation. 2005; 112(15):2276-85.
30. Riad A, Westermann D, Escher F, Becher P M, Savvatis K, Lettau O, Heimesaat M M, Bereswill S, Volk H D, Schultheiss H P,Tschope C. Myeloid differentiation factor-88 contributes to TLR9-mediated modulation of acute coxsackievirus B3-induced myocarditis in vivo. Am J Physiol Heart Circ Physiol. 2010; 298(6):H2024-31.
31. Visintin A, Mazzoni A, Spitzer J A,Segal D M. Secreted MD-2 is a large polymeric protein that efficiently confers lipopolysaccharide sensitivity to Toll-like receptor 4. Proc Natl Acad Sci U S A. 2001; 98(21):12156-61.
32. Shimazu R, Akashi S, Ogata H, Nagai Y, Fukudome K, Miyake K,Kimoto M. MD-2, a molecule that confers lipopolysaccharide responsiveness on Toll-like receptor 4. J Exp Med. 1999; 189(11):1777-82.
33. Ren W, Hu L, Hua F, Jin J, Wang Y,Zhu L. Myeloid differentiation protein 2 silencing decreases LPS-induced cytokine production and TLR4/MyD88 pathway activity in alveolar macrophages. Immunol Lett. 2011; 141(1):94-101.
34. Erridge C. Endogenous ligands of TLR2 and TLR4: agonists or assistants? J Leukoc Biol. 2010; 87(6):989-99.
35. Calvano S E, Xiao W, Richards D R, Felciano R M, Baker H V, Cho R J, Chen R O, Brownstein B H, Cobb J P, Tschoeke S K, Miller-Graziano C, Moldawer L L, Mindrinos M N, Davis R W, Tompkins R G,Lowry S F. A network-based analysis of systemic inflammation in humans. Nature. 2005; 437(7061):1032-7.
36. Gioannini T L, Teghanemt A, Zhang D, Coussens N P, Dockstader W, Ramaswamy S,Weiss J P. Isolation of an endotoxin-MD-2 complex that produces Toll-like receptor 4-dependent cell activation at picomolar concentrations. Proc Natl Acad Sci U S A. 2004; 101 (12):4186-91.
37. Pugin J, Stern-Voeffray S, Daubeuf B, Matthay M A, Elson G,Dunn-Siegrist I. Soluble MD-2 activity in plasma from patients with severe sepsis and septic shock. Blood. 2004; 104(13):4071-9.
38. Frazier W J, Xue J, Luce W A,Liu Y. MAPK signaling drives inflammation in LPS-stimulated cardiomyocytes: the route of crosstalk to G-protein-coupled receptors. PLoS One. 2012; 7(11):e50071.
39. Maisel A S, Krishnaswamy P, Nowak R M, Mccord J, Hollander J E, Duc P, Omland T, Storrow A B, Abraham W T, Wu A H, Clopton P, Steg P G, Westheim A, Knudsen C W, Perez A, Kazanegra R, Herrmann H C,Mccullough P A. Rapid measurement of B-type natriuretic peptide in the emergency diagnosis of heart failure. N Engl J Med. 2002; 347(3):161-7.
40. De Lemos J A, Mcguire D K,Drazner M H. B-type natriuretic peptide in cardiovascular disease. Lancet. 2003; 362(9380):316-22.
41. Troughton R W, Frampton C M, Yandle T G, Espiner E A, Nicholls M G,Richards A M. Treatment of heart failure guided by plasma aminoterminal brain natriuretic peptide (N-BNP) concentrations. Lancet. 2000; 355(9210):1126-30.

## Claims

1. Method for determining the risk of mortality in a proband, especially a heart failure proband, **characterized in that** the proband's level of myeloid differentiation factor 2 (MD-2) is determined and in case of a higher MD-2 level a higher risk of mortality is diagnosed.

2. Method for determining the risk of mortality in a proband according to claim 1, **characterized in that** the MD-2 level is determined in vitro based on body material samples, especially body fluid samples or body tissue samples.

3. Method for determining the risk of mortality in a proband according to claim 1 or 2, **characterized in that** the body fluid samples are samples of blood plasma, blood serum and/or cerebrospinal fluid, preferably blood serum samples.

4. Method for determining the risk of mortality in a proband according to any one of claims 1-3, **characterized in that** the heart failure is dilated cardiomyopathy (DCM).

5. Method for determining the risk of mortality in a proband according to any one of claims 1-4, **characterized in that** the proband is an eucariote, preferably selected from human or animal, most preferably a human proband.

6. Method for determining the risk of mortality in a proband according to any one of claims 1-5, **characterized in that** the MD-2 is analyzed on protein level or on nucleic acid level, preferably on protein level.

7. Method for determining the risk of mortality in a proband, especially a heart failure proband, according to any one of claims 1-6, **characterized in that** additionally the brain natriuretic peptide (BNP) level and/or the amino-terminal pro-brain natriuretic peptide (nt-pro BNP) level is determined and in case of MD-2 level and BNP level and/or nt-pro BNP level being higher a higher risk of mortality is diagnosed.

8. Method for determining the risk of mortality in a proband according to claim 7, **characterized in that** the BNP- and/or nt-pro BNP-level is/are determined in vitro based on body material samples, especially body fluid samples or body tissue samples.

9. Method for diagnosing or pre-diagnosing a heart failure disease or for determining the risk of a proband to develop a heart failure disease, **characterized in that** the proband's level of myeloid differentiation factor 2 (MD-2) is determined and compared to a MD-2 level which is associated with no heart failure (of a healthy proband), whereas in case of a higher MD-2 level heart failure or an increased risk of developing a heart failure is diagnosed/pre-diagnosed.

10. Diagnostic kit for carrying out a method according to any one of claims 1-9.
